# EUROPEAN PATENT APPLICATION

(11) **EP 3 278 789 A1**
(43) Date of publication of application: **07.02.2018**
(21) Application number: 16773300.5
(22) Date of filing: 03.03.2016
(51) Int. Cl.: A61K 8/06, A61K 8/34, A61K 8/49, A61Q 19/00, A61K 8/60, A61K 8/02

(54) **COSMETIC COMPOSITION WITH MULTIPLE EMULSION FORMULATION HAVING LAMELLAR LIQUID CRYSTAL STRUCTURE**

(30) Priority: 31.03.2015 KR 20150045233
(71) Applicant: Amorepacific Corporation, Seoul 04542 (KR)
(72) Inventor: KIM, Sun Young, Yongin-si Gyeonggi-do 17074 (KR); CHAE, Byung Guen, Yongin-si Gyeonggi-do 17074 (KR); PARK, Sung Il, Yongin-si Gyeonggi-do 17074 (KR); KANG, Byung Young, Yongin-si Gyeonggi-do 17074 (KR)
(74) Representative: dompatent von Kreisler Selting Werner - Partnerschaft von Patent- und Rechtsanwälten mbB
(86) International application number: PCT/KR2016/002120
(87) International publication number: WO 2016/159519

(57) **Abstract**

The present invention relates to a cosmetic composition with a multiple emulsion formulation having a lamellar liquid crystal structure, and a method for preparing the same. More particularly, the cosmetic composition is prepared using different single emulsification type emulsions, i.e. a W/O type emulsion and an O/W type emulsion which has a lamellar liquid crystal structure, or using a mixture having constituent ingredients thereof. Thus, the present invention has effects of stably maintaining effective ingredients of the cosmetic composition, and enhancing the feeling of use.

## Description

### [Technical Field]

This application claims priority to and the benefit of Korean Patent Application No. 10-2015-0045233, filed on March 31, 2015 in the Korean Intellectual Property Office, the disclosure of which is incorporated herein by reference in their entirety.

The present invention relates to a cosmetic composition of a multiple emulsion formulation having a lamellar liquid crystal structure, and a method for preparing the same.

### [Background Art]

An emulsion is one of formulations that have been widely used for cosmetics to take care of beautiful skin. An emulsion generally refers to a state in which one of two liquids which are immiscible with each other is dispersed in the other liquid in a small particle phase. In general, water and an oil ingredient are used as the two immiscible liquids. In this case, there are an oil-in-water-type (hereinafter referred to as "O/W-type") emulsion in which an oil is dispersed in water, and a water-in-oil-type (hereinafter referred to as "W/O-type") emulsion in which an aqueous ingredient such as water is dispersed in an oil, depending on a dispersed state. A conventional emulsion refers to such a single-emulsification-type emulsion.

With the recent increasing interest in functional cosmetic materials, there is an increasing demand for cosmetic materials of a multiple emulsion formulation such as a water-in-oil-in-water-type (hereinafter referred to as "W/O/W-type") multiple emulsion or an oil-in-water-in-oil-type (hereinafter referred to as "O/W/O-type") multiple emulsion, in which characteristics of the O/W-type emulsion and the W/O-type emulsion as the single-emulsification-type emulsions coexist.

However, the multiple emulsions have drawbacks in that stability of a formulation may be degraded because interfacial coalescence in which interfaces between particles or in particles are damaged often occurs, and an active ingredient may be easily denatured. In particular, because cosmetic materials of the multiple emulsion formulation are used for products having a high moisturizing and property and high nutritionality such as senior creams, it is very important to secure technology of preparing a stable multiple emulsion in order to further improve the quality of products and sufficiently provide an effect of the products to users.

Accordingly, the present inventors have gone through many trials and errors and taken a great amount of effort to find a method in which a lamellar liquid crystal structure is used to stabilize a multiple emulsion formulation. According to the present invention, an emulsifying agent and adjuvant may be used as a means for forming a lamellar liquid crystal structure by properly adjusting conditions for performing each preparation step, and types of the emulsifying agent and adjuvant used.

### [Prior-art Documents]

Multi-Lamellar Emulsion (MLE) for Stabilizing Dermatologically Useful Ingredients and External Base Preparations for General Skin Diseases Utilizing the Same (Korean Patent Publication No. 2002-0070154)
Multilamellar Emulsion Cosmetics Containing Pseudoceramides (Registered Korean Patent No. 10-0527346)

### [Disclosure]

### [Technical Problem]

Therefore, it is an object of the present invention to improve stability of a cosmetic composition by means of a multiple emulsion formulation having a lamellar liquid crystal structure, thereby continuously releasing an active ingredient in the composition at a constant rate.

It is another object of the present invention to further maximize an effect of coexistence of intrinsic senses of feeling in use of a water-in-oil-type (W/O-type) emulsion and an oil-in-water-type (O/W-type) emulsion by means of the stable multiple emulsion formulation.

### [Technical Solution]

To achieve the above objects, one aspect of the present invention provides a cosmetic composition of a multiple emulsion formulation having a lamellar liquid crystal structure. The lamellar liquid crystal structure is formed by adding a liquid crystal-forming emulsifying agent and adjuvant.

Another aspect of the present invention provides a method for preparing a cosmetic composition of a multiple emulsion formulation, which includes:
1) preparing a water-in-oil-type (W/O-type) emulsion;
2) preparing an oil-in-water-type (O/W-type) emulsion; and
3) adding the W/O-type emulsion to the O/W-type emulsion, wherein the O/W-type emulsion has a lamellar liquid crystal structure, and the lamellar liquid crystal structure of the O/W-type emulsion is formed using a liquid crystal-forming emulsifying agent and adjuvant.

Still another aspect of the present invention provides a method for preparing a cosmetic composition of a multiple emulsion formulation, which includes:
1') preparing a W/O-type emulsion;
2') adding an emulsifying agent and adjuvant and an oily base solution to an aqueous base solution; and
3') adding the W/O-type emulsion to a mixed composition including the aqueous base solution, the emulsifying agent and adjuvant, and the oily base solution, wherein the emulsifying agent and adjuvant are a liquid crystal-forming emulsifying agent and adjuvant.

### [Advantageous Effects]

The cosmetic composition of a multiple emulsion formulation having a lamellar liquid crystal structure according to the present invention exhibits high stability due to low coalescence between particles or between interfaces in particles, and has an enhanced sense of feeling in use due to a decrease in precipitation of crystals caused when the multiple emulsion formulation has low stability, and a maximized effect of coexistence of intrinsic senses of feeling in use of a W/O-type emulsion and an O/W-type emulsion.

### [Description of Drawings]

FIG. 1(A) is a polarizing microscope image of particles of a ((W/O)+O)/W-type multiple emulsion having a lamellar liquid crystal structure prepared in Example 1 of the present invention, and (B) is an enlarged image of FIG. 1(A).
FIG. 2(A) is a polarizing microscope image of a lamellar liquid crystal structure formed in the multiple emulsion prepared in Example 1 of the present invention, and (B) is an enlarged image of FIG. 2(A).
FIG. 3 shows results of observing particles of a W/O/W-type multiple emulsion having a lamellar liquid crystal structure prepared in Example 2 of the present invention.
FIG. 4 is a polarizing microscope image of particles of a W/O/W-type multiple emulsion having a lamellar liquid crystal structure prepared in Example 3 of the present invention: (A) and (B) are enlarged images with magnifications of 50× and 20×, respectively.
FIG. 5 is a polarizing microscope image of the lamellar liquid crystal structure of the multiple emulsion prepared in Example 3 of the present invention: (A) and (B) are enlarged images with magnifications of 50× and 20×, respectively.
FIG. 6 is a graph plotted for results obtained by determining a change according to time of the multiple emulsion prepared in Example 1 for 12 weeks immediately after preparation of the multiple emulsion.
FIG. 7 is a graph plotted for results obtained by determining a change according to time of the multiple emulsion prepared in Example 2 for 4 weeks immediately after preparation of the multiple emulsion.
FIG. 8 shows results of observing a particle state and stability of a composition for 4 weeks elapsed since the multiple emulsion prepared in Example 1 is stored at 60°C, 45°C, refrigeration temperature, freezing temperature, and in cycles (each cycle of -10 to 45°C at an interval of 12 hours) immediately after preparation of the multiple emulsion.
FIG. 9 shows results of observing a particle state and stability of a composition for 12 weeks elapsed since the multiple emulsion prepared in Example 2 is stored at 45°C, refrigeration temperature, freezing temperature, and in cycles (each cycle of -10 to 45°C at an interval of 12 hours) immediately after preparation of the multiple emulsion.

### [Best Mode]

Hereinafter, the present invention will be described in further detail.

The present invention provides a cosmetic composition of a multiple emulsion formulation having a lamellar liquid crystal structure, and a method for preparing the same.

According to exemplary embodiments of the present invention, when the lamellar liquid crystal structure may be formed form a liquid crystal-forming emulsifying agent and adjuvant, all types of the liquid crystal-forming emulsifying agent and adjuvant may fall within the scope of the present invention. However, according to one of preparation methods provided in the following examples, the present invention may be more effectively put into practice.

The following Examples 1 to 3 are disclosed as the most representative examples to aid in understanding the present invention. Therefore, it should be understood that the scope of the present invention is not limited to the examples thereof, but indented to cover all modifications and equivalents within the scope of the appended claims.

Throughout this specification, the unit "%" refers to '% by weight.'

### [Method of preparing multiple emulsion having lamellar liquid crystal structure]

### <Example 1>

According to one exemplary embodiment of the present invention, a water-in-oil-type (W/O-type) emulsion may be mixed with an oil-in-water-type (O/W-type) emulsion having a lamellar liquid crystal structure to prepare a multiple emulsion (hereinafter referred to as a ((water-in-oil) + oil)-in-water-type [((W/O)+O)/W-type] multiple emulsion) in which O/W-type emulsion particles and O/W/O-type emulsion particles co-exist. The multiple emulsion has a lamellar liquid crystal structure.

More specifically, a method for preparing the multiple emulsion may include:
1) preparing a W/O-type emulsion;
2) preparing an O/W-type emulsion; and
3) adding the W/O-type emulsion to the O/W-type emulsion. In this case, the O/W-type emulsion has a lamellar liquid crystal structure, and the lamellar liquid crystal structure of the O/W-type emulsion is formed by a liquid crystal-forming emulsifying agent and adjuvant. Also, a lamellar liquid crystal structure of multiple emulsion particles is formed from the liquid crystal-forming emulsifying agent and adjuvant.

The method for preparing the multiple emulsion may further include adding a thickening agent after Step 3) of adding the W/O-type emulsion.

Ingredients constituting the W/O-type and O/W-type emulsions of Steps 1) to 3) are listed in the following Tables 1 and 2.

**[Table 1]**

| W/O-type emulsions | | | |
|---|---|---|---|
| Items | Ingredient name (% by weight) | Preparative Example 1 | Preparative Example 2 |
| Aqueous base | Water | Proper amount | Proper amount |
| | Preservative | Proper amount | Proper amount |
| | Disodium EDTA | Proper amount | Proper amount |
| | Glycerin | 10 | - |
| | Butylene glycol | - | 10 |
| Emulsifying agent and adjuvant | PEG-10 dimethicone | 2 | 2 |
| | Disteardimonium hectorite | 0.9 | 0.9 |
| | PEG-9 polydimethylsiloxyethyl dimethicone | 1.5 | 1.5 |
| | Cetyl PEG/PPG-10/1 dimethicone | 1.0 | 1.0 |
| Oily base | Silicone oil | 18 | 18 |
| | Oil | 9 | 9 |

**[Table 2]**

| O/W-type emulsions | | | | | |
|---|---|---|---|---|---|
| Items | Ingredient name (% by weight) | Preparative Example 1 | Preparative Example 2 | Preparative Example 3 | Preparative Example 4 |
| Aqueous base | Water | Proper amount | Proper amount | Proper amount | Proper amount |
| | Preservative | Proper amount | Proper amount | Proper amount | Proper amount |
| | Disodium EDTA | Proper amount | Proper amount | Proper amount | Proper amount |
| | Glycerin | - | 5 | - | 5 |
| | Butylene glycol | 10 | 5 | 10 | 5 |
| Emulsifying agent and adjuvant | Arachidyl glucoside | 0.75 | - | 0.75 | - |
| | Arachidyl alcohol | 2.75 | - | 2.75 | - |
| | Behenyl alcohol | 1.5 | - | 1.5 | - |
| | Cetearyl glucoside | - | 1 | - | 1 |
| | Cetearyl alcohol | - | 4 | - | 4 |
| Oily base | Squalane | 5 | 5 | - | - |
| | Cetyl ethylhexanoate | 5 | 5 | - | - |
| | Octyl dodecyl myristate | - | - | 5 | 5 |
| Thickening agent | | Proper amount | Proper amount | Proper amount | Proper amount |
| Perfume | | Proper amount | Proper amount | Proper amount | Proper amount |

### Aqueous base

The aqueous base includes distilled (DI) water and a water-soluble polyhydric alcohol, and may include other water-soluble active ingredients.

The polyhydric alcohol may include one or more selected from the group consisting of propylene glycol, butylene glycol, dipropylene glycol, glycerin, diglycerin, polyglycerin, erythritol, pentaerythritol, sorbitan, glucose, sorbitol, trehalose, and polyethylene glycol, but the present invention is not limited thereto. Preferably, the polyhydric alcohol may be glycerin or 1,3-butylene glycol, as listed in Table 1 or 2.

The aqueous base may further include 1,1-diphenyl-2-picrylhydrazyl (DPPH) to measure a SC₅₀ value as a measure for evaluating an active oxygen removal function.

### Oily base

Throughout this specification, the term 'oily base' refers to an oil-soluble ingredient rather than the ingredients of the emulsifying agent and adjuvant.

The oily base includes an oil. The oil may include one or more selected from a hydrocarbon oil, an ester oil, a natural oil, and silicone oil, but the present invention is not limited thereto.

More specifically, the hydrocarbon oil may include squalane, squalene, liquid paraffin, paraffin, isoparaffin, and the like, the ester oil may include one or more selected from ethylhexyl isononanoate, ethylhexyl isopalmitate, ethylhexyl isostearate, ethylhexyl myristate, ethylhexyl neopentanoate, ethylhexyl oleate, ethylhexyl palmitate, ethylhexyl stearate, and the like, and the natural oil may include olive oil, avocado oil, castor oil, jojoba oil, soybean oil, corn oil, chamomile oil, triglycerin, trioctanoic acid glycerin, and the like.

Preferably, the oily base may be dimethicone, cyclopentasiloxane, cyclohexasiloxane, squalane, triethylhexanoin, or cetyl ethylhexanoate, as listed in Table 1 or 2.

### Emulsifying agent and adjuvant

As an emulsifying agent and adjuvant used to prepare a W/O-type emulsion, one or more selected from the group consisting of PEG-10 dimethicone, disteardimonium hectorite, PEG-9 polydimethylsiloxyethyl dimethicone, cetyl PEG/PEG-10/1 dimethicone, and a combination thereof may be preferably used, but the present invention is not limited thereto.

The emulsifying agent and adjuvant used for the preparing of an O/W-type emulsion is a liquid crystal-forming emulsifying agent and adjuvant, and is one or more selected from compounds represented by the following Formulas 1, 2 and 3: 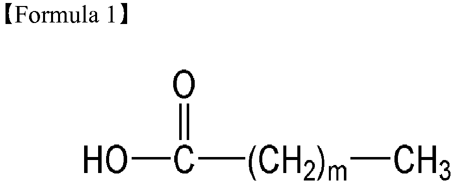 wherein m is an integer in a range of 11 ≤ m ≤ 21; 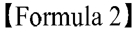 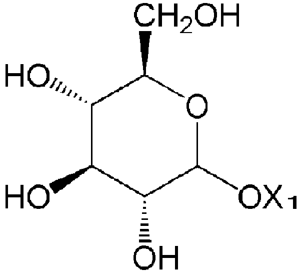 wherein X₁ is a dehydrogenated C11 to C21 aliphatic alcohol;and 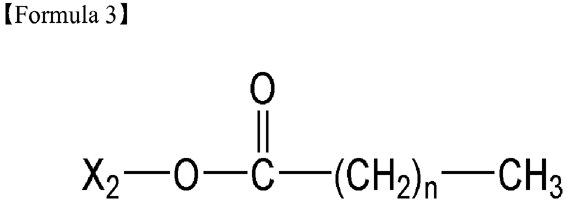 wherein X₂ is one selected from the group consisting of dehydrogenated sorbitan, dehydrogenated glycerin, dehydrogenated polyglycerin, dehydrogenated sucrose, dehydrogenated cetearyl alcohol, polyethylene glycol, polyethylene glycol glyceryl, polyethylene glycol sorbitan, and sorbitan, and n is an integer in a range of 10 ≤ n ≤18.

For Formulas 1 to 3, more specifically, the compound of Formula 1 may be an aliphatic alcohol that may include arachidyl alcohol, behenyl alcohol, cetyl alcohol, stearyl alcohol, and cetearyl alcohol.

The compound of Formula 2 is an alkyl glucoside including a dehydrogenated aliphatic alcohol. In this case, the aliphatic alcohol may include arachidyl alcohol, behenyl alcohol, cetyl alcohol, stearyl alcohol, and cetearyl alcohol. Preferably, the compound of Formula 2 may be arachidyl glucoside or cetearyl glucoside.

The compound of Formula 3 is a fatty acid ester that may include a sorbitan fatty acid ester, a glycerin fatty acid ester, and a sucrose fatty acid ester. More specifically, the sorbitan fatty acid ester may include sorbitan laurate, sorbitan stearate, and sorbitan olebate, the glycerin fatty acid ester may include glyceryl stearate, and polyglyceryl-10 stearate, and the sucrose fatty acid ester may include sucrose polystearate.

Also, in addition to the compounds of Formulas 1 to 3, the liquid crystal-forming emulsifying agent and adjuvant according to the present invention may be hydrogenated lecithin.

Preferably, one or more selected from arachidyl alcohol, behenyl alcohol, cetyl alcohol, stearyl alcohol, cetearyl alcohol, arachidyl glucoside, cetearyl glucoside, sorbitan laurate, sorbitan stearate, glyceryl stearate, sucrose polystearate, and hydrogenated lecithin may be selected as the liquid crystal-forming emulsifying agent and adjuvant according to the present invention. More preferably, one or more selected from arachidyl alcohol, behenyl alcohol, arachidyl glucoside, cetearyl alcohol, and cetearyl glucoside may be selected as the liquid crystal-forming emulsifying agent and adjuvant according to the present invention.

The liquid crystal-forming emulsifying agent and adjuvant used to prepare the O/W emulsion in this Example 1 include arachidyl alcohol, behenyl alcohol, cetearyl alcohol, arachidyl glucoside, and cetearyl glucoside.

Any types of emulsifying agents and adjuvants with which liquid crystals are easily formed may be used without limitation as the liquid crystal-forming emulsifying agent and adjuvant. In this case, the liquid crystal-forming emulsifying agent and adjuvant serves to cause steric hindrance between particles of the multiple emulsion to secure stability between particles and particles and between interfaces and interfaces.

### Thickening agent

To increase a viscosity of a multiple emulsion formulation, a thickening agent may be added with a polyhydric alcohol. In this case, one or more selected from hydroxyethyl acrylate/sodium acryloyldimethyl taurate copolymer, polyglyceryl methacrylate/propylene glycol, xanthan gum and carboxyvinyl polymer, and carbomer may be selected as the thickening agent, but the present invention is not limited thereto.

Table 3 show Preparative Examples #1 to #8 in which emulsions are formed according to the stepwise processes described in this Example 1 to compare conditions for forming emulsion and stabilities of emulsions formed thereby.

**[Table 3]**

| Preparative Examples | W/O content (% by weight) | W/O temperature | W/O input conditions | Results |
|---|---|---|---|---|
| #1 | 5 | 65°C | 65°C, 4,000 rpm, and 2 minutes | Satisfactory emulsion formation |
| #2 | 10 | 50°C | 65°C, 7,000 rpm, and 2 minutes | Increase in viscosity and occurrence of phase inversion during mixing |
| #3 | 15 | 50°C | 50°C, 4,000 rpm, and 2 minutes | Satisfactory emulsion formation |
| #4 | 15 | 50°C | 75°C, 4,000 rpm, and 2 minutes | Increase in viscosity and occurrence of phase inversion during mixing |
| #5 | 20 | 50°C | 60°C, 5,500 rpm, and 2 minutes | Satisfactory emulsion formation |
| #6 | 20 | 70°C | 60°C, 4,000 rpm, and 2 minutes | Increase in viscosity and occurrence of phase inversion during mixing |
| #7 | 25 | 50°C | 60°C, 6,500 rpm, and 2 minutes | Satisfactory emulsion formation |
| #8 | 30 | 50°C | 55°C, 4,000 rpm, and 2 minutes | Emulsion with rough surface |

Hereinafter, the method for preparing a ((W/O)+O)/W-type multiple emulsion having a lamellar liquid crystal structure according to one exemplary embodiment of the present invention will be described in detail.

### 1) Preparing W/O-type emulsion

A W/O-type emulsion may be prepared by stirring or homogeneously mixing components of the W/O-type emulsion listed in Table 1 using a paddle mixer. A temperature of the W/O-type emulsion is preferably maintained in a range of 40 to 70°C, more preferably 50 to 65°C.

### 2) Preparing O/W-type emulsion

An O/W-type emulsion may be prepared by adding an oily base having a temperature of 75°C, and a liquid crystal-forming emulsifying agent and adjuvant to an aqueous base heated to 70°C, and stirring the resulting mixture using a homomixer. An rpm value set in the homomixer is a value typically used during stirring in the related art.

The prepared O/W-type emulsion has a lamellar liquid crystal structure, and the lamellar liquid crystal structure is formed by adding the liquid crystal-forming emulsifying agent and adjuvant.

### 3) Adding W/O-type emulsion to O/W-type emulsion

This step includes preparing a multiple emulsion.

A content of the W/O-type emulsion added is preferably in a range of 1 to 30% by weight, more preferably 5 to 25% by weight. By comparing Preparative Examples #3 and #8 listed in Table 3, it can be seen that the multiple emulsion is formed satisfactorily when the W/O-type emulsion is added at a content of 15% by weight (# 3), but the multiple emulsion with a rough surface is formed when the W/O-type emulsion is added at a content of 25% by weight or more (# 8).

A homomixer may be used to perform the addition and stirring, and an rpm value set in the homomixer is in a range that may be typically set in the related art in consideration of the temperature and content of the emulsion added. Preferably, the rpm value is in a range of 3,000 to 8,000 rpm, more preferably 4,000 to 6,500 rpm. In this case, a stirring time is in a range of 2 to 5 minutes.

Also, a temperature of the W/O-type emulsion when added is preferably maintained in a range of 50 to 65°C. By comparing Preparative Examples #5 and #6 listed in Table 3, it can be seen that the multiple emulsion is formed satisfactorily when the temperature of the W/O-type emulsion is maintained at 50°C (#5), but a viscosity of the multiple emulsion formulation increases and phase inversion occurs during stirring when the temperature of the W/O-type emulsion is maintained at 70°C (#6).

After Step 3) of this Example 1, a thickening agent may be additionally added to enhance a sense of feeling in use of the multiple emulsion. After the thickening agent is added, the resulting mixture is preferably stirred at 4,000 to 6,500 rpm for another 2 to 5 minutes using the homomixer.

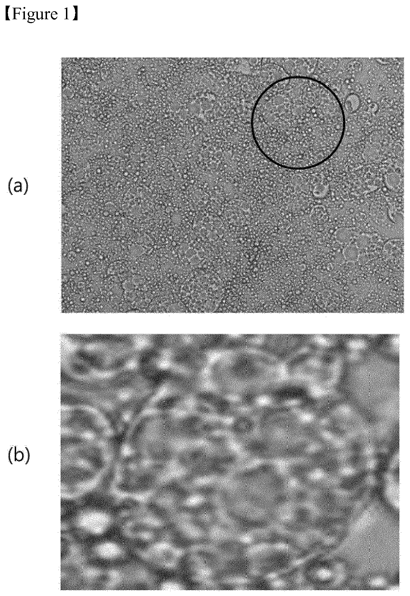

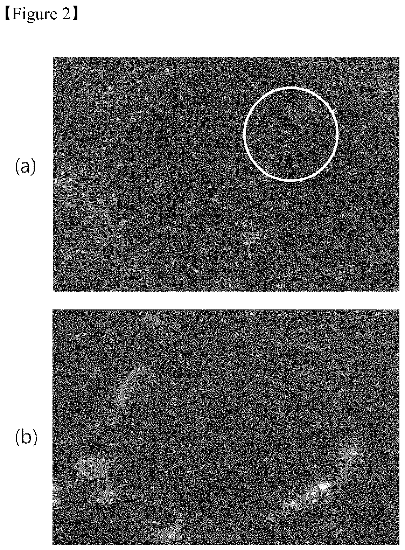

The lamellar liquid crystal structure of the ((W/O)+O)/W-type multiple emulsion having a lamellar liquid crystal structure (FIG. 1) prepared in this Example 1 was verified using a polarizing microscope (FIG. 2).

In this Example 1, when the 'W/O-type emulsion' and 'O/W-type emulsion' are mutually replaced during the process steps, an O/W/O-type multiple emulsion having a lamellar liquid crystal structure may be prepared.

### <Example 2>

According to another exemplary embodiment of the present invention, an oily base solution, a liquid crystal-forming emulsifying agent and adjuvant, and a W/O-type emulsion may be added to an aqueous base solution to prepare a W/O/W-type multiple emulsion having a lamellar liquid crystal structure.

More specifically, a method for preparing the W/O/W-type multiple emulsion may include:
1') preparing a W/O-type emulsion;
2') adding an emulsifying agent and adjuvant and an oily base to an aqueous base; and
3') adding the W/O-type emulsion to the aqueous base to which the emulsifying agent and adjuvant and the oily base are added. In this case, the emulsifying agent and adjuvant are a liquid crystal-forming emulsifying agent and adjuvant, and the liquid crystal-forming emulsifying agent and adjuvant serve to form a lamellar liquid crystal structure of the multiple emulsion.

The method for preparing the W/O/W-type multiple emulsion may further include adding a thickening agent after Step 3') of adding the W/O-type emulsion.

Ingredients constituting the W/O-type emulsion, the aqueous base solution, the emulsifying agent and adjuvant, the oily base solution, and the thickening agent used in Steps 1') to 3') are listed in the following Tables 4 and 5. The following components are as described above in Example 1, and thus a detailed description thereof is omitted.

**[Table 4]**

| W/O-type emulsions | | | |
|---|---|---|---|
| Items | Ingredient name (% by weight) | Preparative Example 1 | reparative Example 2 |
| Aqueous base | Water | Proper amount | Proper amount |
| | Preservative | Proper amount | Proper amount |
| | Disodium EDTA | Proper amount | Proper amount |
| | Glycerin | 10 | - |
| | Butylene glycol | - | 10 |
| Emulsifying agent and adjuvant | PEG-10 dimethicone | 2 | 2 |
| | Disteardimonium hectorite | 0.9 | 0.9 |
| | PEG-9 polydimethylsiloxyethyl dimethicone | 1.5 | 1.5 |
| | Cetyl PEG/PPG-10/1 dimethicone | 1.0 | 1.0 |
| Oily base | Silicone oil | 18 | 18 |
| | Oil | 9 | 9 |

**[Table 5]**

| O/W-type emulsions | | | | | |
|---|---|---|---|---|---|
| Items | Ingredient name (% by weight) | Preparative Example 1 | Preparative Example 2 | Preparative Example 3 | Preparative Example 4 |
| Aqueous base | Water | Proper amount | Proper amount | Proper amount | Proper amount |
| | Preservative | Proper amount | Proper amount | Proper amount | Proper amount |
| | Disodium EDTA | Proper amount | Proper amount | Proper amount | Proper amount |
| | Glycerin | - | 5 | - | 5 |
| | Butylene glycol | 10 | 5 | 10 | 5 |
| Emulsifying agent and adjuvant | Arachidyl glucoside | 0.75 | - | 0.75 | - |
| | Arachidyl alcohol | 2.75 | - | 2.75 | - |
| | Behenyl alcohol | 1.5 | - | 1.5 | - |
| | Cetearyl glucoside | - | 1 | - | 1 |
| | Cetearyl alcohol | - | 4 | - | 4 |
| Oily base | Squalane | 5 | 5 | - | - |
| | Cetyl ethylhexanoate | 5 | 5 | - | - |
| | Octyl dodecyl myristate | - | - | 5 | 5 |
| Thickening agent | | Proper amount | Proper amount | Proper amount | Proper amount |
| Perfume | | Proper amount | Proper amount | Proper amount | Proper amount |

Table 6 show Preparative Examples #1 to #5 in which emulsions are formed according to the stepwise processes described in this Example 2 to compare conditions for forming emulsion and stabilities of emulsions formed thereby.

**[Table 6]**

| Preparative Examples | W/O content (% by weight) | W/O temperature | W/O input conditions | W/O/W emulsification conditions | Results |
|---|---|---|---|---|---|
| #1 | 5 | 65°C | Paddle-/homo-mixed at 60 to 65°C and 2,000 rpm | 60 to 65°C, 4,000 to 5,000 rpm, 2 minutes | Satisfactory emulsion formation |
| #2 | 10 | 50°C | Paddle-/homo-mixed at 60 to 65°C and 2,000 rpm | 60 to 65°C, 8,000 rpm, 2 minutes | Increase in viscosity and occurrence of phase inversion during mixing |
| #3 | 15 | 50°C | Paddle-/homo-mixed at 70 to 75°C and 2,000 rpm | 70 to 75°C, 4,000 to 5,000 rpm, 2 minutes | Increase in viscosity and occurrence of phase inversion during mixing |
| #4 | 15 | 50°C | Paddle-/homo-mixed at 55 to 60°C and 2,000 rpm | 55 to 60°C, 6000 rpm, 2 minutes | Satisfactory emulsion formation |
| #5 | 20 | 50°C | Paddle-/homo-mixed at 60 to 65°C and 2,000 rpm | 60 to 65°C, 4,000 to 5,000 rpm, 2 minutes | Increase in viscosity and occurrence of phase inversion during mixing |

Hereinafter, the method for preparing a W/O/W-type multiple emulsion having a lamellar liquid crystal structure according to one exemplary embodiment of the present invention will be described in detail.

### 1') Preparing W/O-type emulsion

A W/O-type emulsion may be prepared by stirring components listed in Table 4 using a paddle mixer or a homomixer. A temperature of the W/O-type emulsion is preferably maintained in a range of 45 to 70°C, more preferably 50 to 65°C.

### 2') adding emulsifying agent and adjuvant and oily base solution to aqueous base solution

An emulsifying agent and adjuvant and an oily base solution are added using a paddle mixer or a homomixer in which a speed of revolution is set to a low rpm value in a state in which a temperature of each of the aqueous base solutions listed in Table 5 is maintained at 65°C. In this case, the emulsifying agent and adjuvant are a liquid crystal-forming emulsifying agent and adjuvant. A temperature of the oily base solution is preferably 75°C, but the present invention is not essentially limited thereto.

The components added in this Step 2') are the same as the ingredients added to prepare the O/W-type emulsion in Step 2) of Example 1. However, after the components are added, stirring the mixture of this Step 2') to emulsify the mixture is not performed, unlike Example 1. Therefore, there is a difference in that the mixture is maintained in a simple mixture state without forming an W/O -type emulsion having a lamellar liquid crystal structure.

### 3') Adding the W/O-type emulsion to aqueous base solution to which emulsifying agent and adjuvant and oily base are added

The W/O-type emulsion is added without delay after the emulsifying agent and adjuvant and the oily base are added to the aqueous base.

In this case, a temperature of the W/O-type emulsion is preferably in a range of 50 to 65°C, and a content of the W/O-type emulsion added is in a range of 5 to 25% by weight, preferably 10 to 15% by weight. A homomixer may be used to perform the addition and stirring, and an rpm value set in the homomixer is in a range that may be typically set in the related art in consideration of the temperature and content of the emulsion added. Preferably, the rpm value is in a range of 3,000 to 8,000 rpm, more preferably 3,000 to 6,500 rpm.

By comparing Preparative Examples #1 and #2 listed in Table 6, it can be seen that the multiple emulsion is formed satisfactorily when the rpm value of the homomixer is in a range of 4,000 to 5,000 rpm (#1), but a viscosity of the multiple emulsion increases and phase inversion occurs during stirring when the rpm value of the homomixer is 8,000 rpm (#2).

After the W/O-type emulsion is added, the mixture is emulsified. In this case, the homomixer may be used without being replaced, and an rpm value set in the homomixer is preferably in a range of 3,000 to 6,500 rpm. During the emulsification, a temperature of the mixture is preferably maintained in a range of 55 to 65°C (Preparative Examples #1 and #4 in Table 6).

After Step 3') of this Example 2, a thickening agent may be additionally added to enhance a sense of feeling in use of the multiple emulsion. After the thickening agent is added, the resulting mixture is stirred at 4,000 to 6,500 rpm for another 2 to 5 minutes using the homomixer.

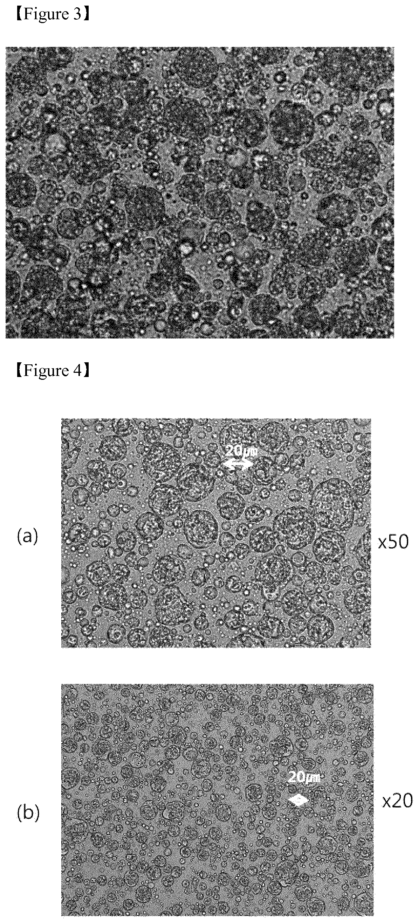

A lamellar liquid crystal structure of the W/O/W-type multiple emulsion formulation (FIG. 3) prepared in this Example 2 may be observed using a polarizing microscope.

In this Example 2, when the 'W/O-type emulsion' and 'O/W-type emulsion' are mutually replaced; and the 'aqueous base solution', and the 'emulsifying agent and adjuvant and the oily base solution' are mutually replaced during the process steps, an O/W/O-type multiple emulsion having a lamellar liquid crystal structure may be prepared.

### <Example 3>

Examples of the present invention will be described in further detail to easily put the present invention into practice. In this Example 3, a cosmetic composition of a multiple emulsion formulation having a lamellar liquid crystal structure according to the present invention is prepared according to the method disclosed in Example 2. Detailed specifications for the cosmetic composition are listed in the following Tables 7 and 8.

**[Table 7]**

| W/O-type emulsions | | |
|---|---|---|
| Items | Ingredient name | Content (% by weight) |
| W/O | | 15 |
| Aqueous base | Water | 8.5875 |
| | Phenoxyethanol | 0.045 |
| | Disodium EDTA | 0.0075 |
| | Glycerin | 1.5 |
| Emulsifying agent and adjuvant | PEG-10 dimethicone | 0.3 |
| | Disteardimonium hectorite | 0.135 |
| | Lauryl PEG-9 polydimethylsiloxyethyl dimethicone | 0.225 |
| | Cetyl PEG/PPG-10/1 dimethicone | 0.15 |
| Oily base | Dimethicone | 0.75 |
| | Cyclopentasiloxane | 1.35 |
| | Cyclohexasiloxane | 0.6 |
| | Triethylhexanoin | 0.45 |
| | Squalane | 0.45 |
| | Butylene glycol dicarprylate/dicaprate | 0.45 |

**[Table 8]**

| Aqueous base solution, emulsifying agent and adjuvant, oily base solution, and thickening agent | | |
|---|---|---|
| Items | Ingredient name | Content (% by weight) |
| Aqueous base solution | | 64.8 |
| Aqueous base | Water | 59.45 |
| | Phenoxyethanol | 0.3 |
| | Disodium EDTA | 0.05 |
| | Butylene glycol | 5 |
| Emulsifying agent and adjuvant (liquid crystal-forming emulsifying agent) | | 5 |
| Emulsifying agent and adjuvant | Arachidyl glucoside | 0.75 |
| | Arachidyl alcohol | 2.75 |
| | Behenyl alcohol | 1.5 |
| Oily base solution | | 10 |
| Oily base | Squalane | 5 |
| | Cetyl ethylhexanoate | 5 |
| Thickening agent | | 5.2 |
| Thickening agent | Hydroxyethyl acrylate/sodium acryloyldimethyl taurate copolymer | 0.2 |
| | Butylene glycol | 5 |

A temperature of the aqueous base solution was set to 55 to 65°C, temperatures of the emulsifying agent and adjuvant and the oily base solution were set to 78°C, and a temperature of the W/O-type emulsion was set to 63°C. The mixture including the aqueous base solution, the emulsifying agent and adjuvant, the oily base solution, and the W/O-type emulsion was emulsified by stirring for 3 minutes using a homomixer while a temperature of the mixture was maintained at 58 to 65°C. In this case, an rpm value set in the homomixer was 4,000 rpm.

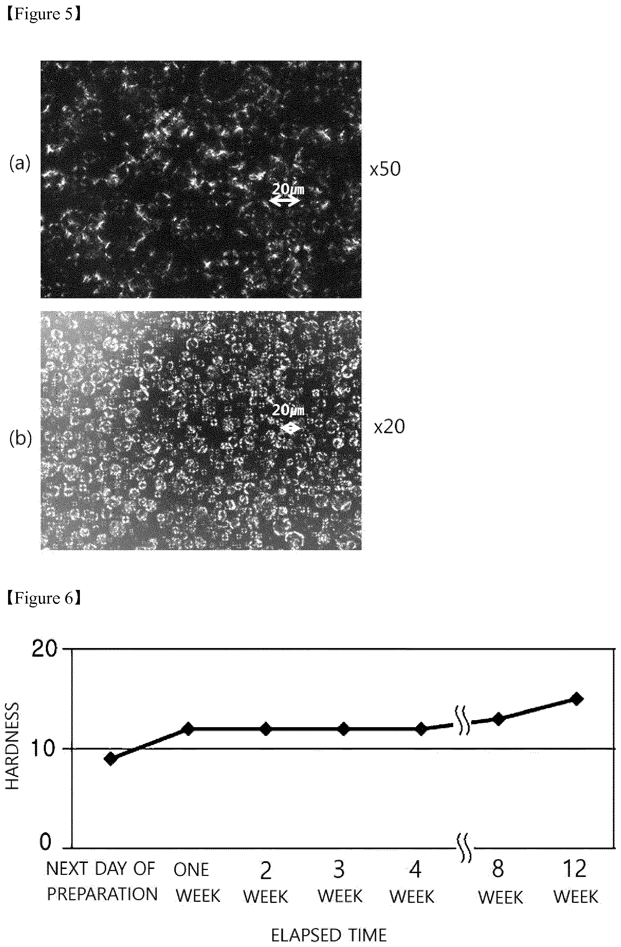

Next, a thickening agent was additionally added to the mixture, and the resulting mixture was stirred at 60°C and 4,000 rpm for another 2 minutes using the homomixer, degased, cooled, and then discharged to prepare a cosmetic composition for a W/O/W-type emulsion formulation having a lamellar liquid crystal structure (FIG. 4). The lamellar liquid crystal structure was observed using a polarizing microscope (FIG. 5).

### [Test for determining stability and change according to time of multiple emulsion formulation having lamellar liquid crystal structure]

### <Experimental Example 1>

To check stabilities of the multiple emulsion formulations having a lamellar liquid crystal structure prepared in Examples 1 and 2, a change according to time of each of the multiple emulsion formulations for 4 weeks after preparation of the multiple emulsion formulations was determined (Sun rheometer CR-500DX, at 30°C). Thereafter, a change according to time of the multiple emulsion of Example 1 for 8 weeks was further determined.

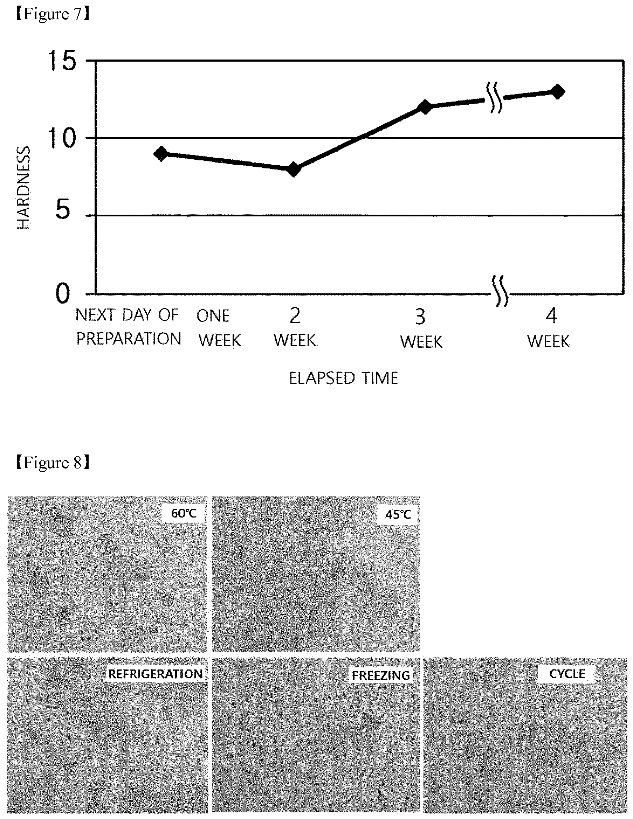

From the results of determining the change according to time for 4 weeks after the preparation, both the multiple emulsion formulations prepared in Examples 1 and 2 had a constant hardness of approximately 9 to 13 N for 4 weeks immediately after the preparation, and thus exhibited a stable pattern in the change according to time (FIGS. 6 and 7). Also, when the multiple emulsion of Example 1 was stored for another 4 weeks to 8 weeks, the multiple emulsion also had almost a constant hardness of 12 to 15 N (FIG. 6).

Therefore, it can be seen that the multiple emulsion formulation having a lamellar liquid crystal structure according to the present invention was a very stable formulation with a small change according to time.

### <Experimental Example 2>

The multiple emulsion prepared in Example 1 was stored at 60°C, 45°C, refrigeration and freezing temperatures, and in cycles (each cycle of -10 to 45°C at an interval of 12 hours) immediately after preparation of the multiple emulsion. After the elapse of 4 weeks, the particle state and stability of the composition were observed (FIG. 8).

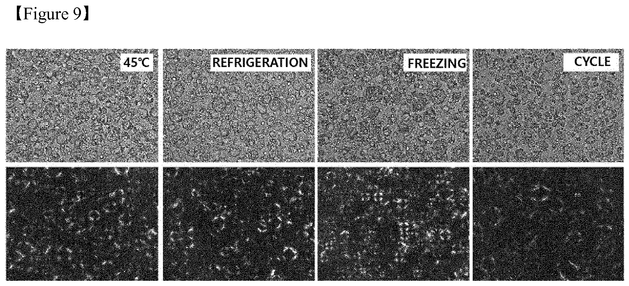

The multiple emulsion prepared in Example 2 was stored at 45°C, refrigeration and freezing temperatures, and in cycles (each cycle of -10 to 45°C at an interval of 12 hours) immediately after preparation of the multiple emulsion. After the elapse of 12 weeks, the particle state and stability of the composition were observed (FIG. 9).

Referring to FIGS. 8 and 9, it can be seen that the multiple emulsion samples stored at each of the given temperatures were generally maintained in a good state as immediately after preparation of the multiple emulsion. In all the samples to be tested, there was no precipitation of crystals caused due to degraded stability of the multiple emulsion formulations, and no ponding phenomenon was also observed.

As can be seen from the results of the observation, the stability of the multiple emulsion is maintained at a high level because the lamellar liquid crystal structure protects the particles in the multiple emulsion from an external environment such as heat, air, or moisture.

## Claims

1. A cosmetic composition of a multiple emulsion formulation having a lamellar liquid crystal structure.

2. The cosmetic composition of claim 1, wherein the lamellar liquid crystal structure is formed by adding a liquid crystal-forming emulsifying agent and adjuvant.

3. The cosmetic composition of claim 2, wherein the liquid crystal-forming emulsifying agent and adjuvant comprise one or more selected from compounds represented by the following Formulas 1, 2, and 3: 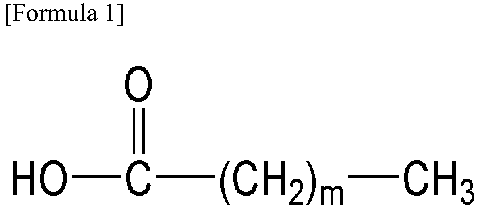 wherein m is an integer in a range of 11 ≤ m ≤ 21; 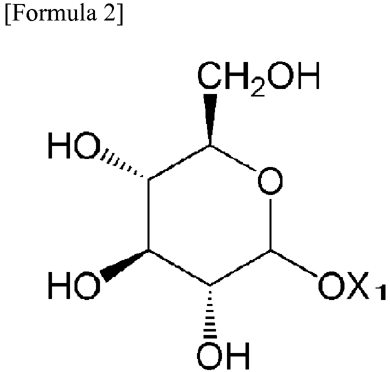 wherein X₁ is a dehydrogenated C11 to C21 aliphatic alcohol; and 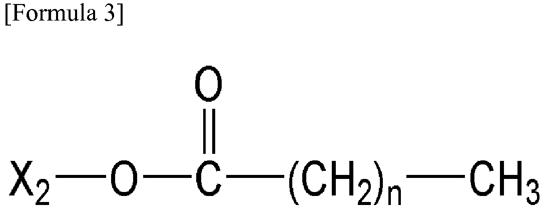 wherein X₂ is one selected from the group consisting of dehydrogenated sorbitan, dehydrogenated glycerin, dehydrogenated polyglycerin, dehydrogenated sucrose, dehydrogenated cetearyl alcohol, polyethylene glycol, polyethylene glycol glyceryl, polyethylene glycol sorbitan, and sorbitan, and n is an integer in a range of 10 ≤ n ≤18.

4. The cosmetic composition of claim 2, wherein the liquid crystal-forming emulsifying agent and adjuvant comprise one or more selected from the group consisting of arachidyl alcohol, behenyl alcohol, cetyl alcohol, stearyl alcohol, cetearyl alcohol, arachidyl glucoside, cetearyl glucoside, sorbitan laurate, sorbitan stearate, glyceryl stearate, sucrose polystearate, hydrogenated lecithin, and a combination thereof.

5. A method for preparing a cosmetic composition of a multiple emulsion formulation, comprising:
preparing a water-in-oil (W/O)-type emulsion;
preparing an oil-in-water (O/W)-type emulsion; and
adding the W/O-type emulsion to the O/W-type emulsion,
wherein the O/W-type emulsion has a lamellar liquid crystal structure, and the lamellar liquid crystal structure of the O/W-type emulsion is formed using a liquid crystal-forming emulsifying agent and adjuvant.

6. The method of claim 5, wherein a temperature of the W/O-type emulsion is maintained in a range of 45 to 65°C.

7. The method of claim 5, wherein the W/O-type emulsion and the O/W-type emulsion is mutually replaced.

8. A method for preparing a cosmetic composition of a multiple emulsion formulation, comprising:
preparing a W/O-type emulsion;
adding an emulsifying agent and adjuvant and an oily base solution to an aqueous base solution; and
adding the W/O-type emulsion to a mixed composition including the aqueous base solution, the emulsifying agent and adjuvant, and the oily base solution,
wherein the emulsifying agent and adjuvant are a liquid crystal-forming emulsifying agent and adjuvant.

9. The method of claim 8, wherein a temperature of the W/O-type emulsion is maintained in a range of 50 to 65°C.

10. The method of claim 8, wherein the W/O-type emulsion and the mixed composition including the aqueous base solution, the emulsifying agent and adjuvant, and the oily base solution are mixed at 1,000 to 3,000 rpm.

11. The method of claim 5 or 8, wherein a content of the W/O-type emulsion added is in a range of 5 to 25% by weight, based on the total weight of the mixed composition.

12. The method of claim 8, wherein the W/O-type emulsion is replaced with an O/W-type emulsion, and the aqueous base solution, and the emulsifying agent and adjuvant and the oily base solution are mutually replaced.

13. The method of claim 5 or 8, further comprising:
adding a thickening agent after the adding of the W/O-type emulsion.

14. The method of claim 5 or 8, further comprising:
determining whether a lamellar liquid crystal structure is formed in the composition after the final step defined in the claims.

15. The method of claim 5 or 8, wherein the liquid crystal-forming emulsifying agent and adjuvant comprise one or more selected from the group consisting of arachidyl alcohol, behenyl alcohol, cetyl alcohol, stearyl alcohol, cetearyl alcohol, arachidyl glucoside, cetearyl glucoside, sorbitan laurate, sorbitan stearate, glyceryl stearate, sucrose polystearate, hydrogenated lecithin, and a combination thereof.
